# EUROPEAN PATENT SPECIFICATION

(11) **EP 3 721 844 B1**
(45) Date of publication and mention of the grant of the patent: **18.01.2023**
(21) Application number: 19168826.6
(22) Date of filing: 12.04.2019
(51) Int. Cl.: A61F 13/42, A61F 13/56, A61F 13/62

(54) **ABSORBENT ARTICLE COMPRISING ATTACHMENT REGIONS**
SAUGFÄHIGER ARTIKEL MIT BEFESTIGUNGSBEREICHEN
ARTICLE ABSORBANT COMPRENANT DES RÉGIONS DE FIXATION

(43) Date of publication of application: 14.10.2020
(73) Proprietor: The Procter & Gamble Company, Cincinnati, OH 45202 (US)
(72) Inventor: ARIZTI, Blanca, 65824 Schwalbach am Taunus (DE)
(74) Representative: P&G Patent Germany

(56) References cited:
- EP-A1- 3 213 727
- US-A1- 2006 244 614
- US-A1- 2012 116 337

## Description

### Field of the Invention

The present invention relates to an absorbent article comprising attachment regions wherein an attachment region is provided for securing a wearable sensing device to the absorbent article.

### Background of the Invention

An absorbent article may be adapted to secure a wearable sensing device to the absorbent article, for example, to the outside (garment facing side) of the absorbent article.

For example an absorbent article may comprise a wetness indicator which is in liquid communication with the absorbent core, and which is visible through the back sheet, from the garment side of the absorbent article. The wetness indicator is typically designed to change colour when urine comes into contact with the wetness indicator. The change in colour of the wetness indicator may be discernable by the human eye, or the colour change may be difficult to distinguish by the human eye alone, or the colour change may even be indistinguishable to the human eye.

A wearable sensing device which is correctly positioned with respect to the wetness indicator can reliably detect changes in colour of the wetness indicator, even to detect small changes in shade, whether or not those colour changes are discernable to the human eye. Information about the state of wetness of the absorbent article can be transmitted from the wearable sensing device to the care-giver, which may inform the care-giver of the need to change the absorbent article.

Typically the absorbent article is a disposable article used for personal hygiene, meaning that the article is disposed of after use and replaced by a fresh article. The wearable sensing device can be reused multiple times by reaffixing it to a fresh absorbent article following a change.

When the wearable sensing device is attached to the absorbent article, the wearable sensing device needs to be securely attached to avoid or minimise the risk of the sensing device being removed unintentionally or the risk of being removed by someone other than the care-giver, for example by the wearer of the wearable sensing device or by a sibling. Minimising the risk of such removal of the wearable sensing device helps to minimise the risk of a hazard of the wearable sensing device being put into the mouth and presenting a choking hazard.

EP 3 213 727, published on September 6th 2017, discloses an absorbent article and a sensor for detecting, for example, the presence of urine in the absorbent core of the article.

There is a need to provide an attachment region on the garment-facing side of an absorbent article, such as a diaper, which is adapted to receive a wearable sensing device, such as a sensor for detecting wetness, movement, environmental conditions etc.

### Summary of the Invention

According to one embodiment the present invention provides an absorbent article comprising topsheet, backsheet and an absorbent core between the topsheet and the backsheet, the article comprising a front waist region, a back waist region, and a central region between the front and back waist regions, and further comprising fastening elements for securing the article around the waist of a wearer, wherein the article comprises at least two attachment regions:
a first attachment region for securing the fastening elements in the waist region, wherein the first attachment region lies in at least a part of the front waist region, the first attachment region comprises first surface comprising outwardly facing fibres, wherein the fastening elements comprise a fastening surface comprising outwardly facing fibres, and wherein the outwardly facing fibres interact to form a releaseable attachment between the fastenening elements and the first attachment region, wherein the interacting outwardly facing fibres comprise hooks and loops; and
a second attachment region for securing a wearable sensing device, the second attachment region being located on the side of the backsheet opposite to the absorbent core, wherein the second attachment region lies in at least a part of the central region between the front and back waist regions and wherein the second attachment region comprises hooks and/or loops;
wherein the first attachment region has a first surface and the second attachment region has a second surface and wherein the first surface is different from the second surface, and wherein the peel force response of the hooks/loops of the first attachment region is greater than or less than the peel force response of the hooks/loops of the second attachment region.

According to another embodiment the present invention also provides a system comprising an absorbent article as described herein and a wearable sensing device, wherein the wearable sensing device comprises a housing, and wherein a part of the housing cooperates with the second attachment region to releasably secure the wearable sensing device to the absorbent article. Preferably the housing is provided with outwardly facing fibres which interact to form a releaseable attachment between the housing of the wearable sensing device and the second attachment region.

### Brief Description of the Drawings

Figure 1 is a top view of an absorbent article according to an embodiment of the present invention in the form of a diaper with some layers partially removed;
Figure 2 is a transversal cross-section of the embodiment of Figure 1 at the crotch area;
Figures 3a to 3c show a piece of equipment for carrying out a test method;
Figure 4 shows another piece of equipment for carrying out a test method.

### Detailed Description of the Invention

Absorbent articles used for personal hygiene typically comprise a topsheet, a backsheet and an absorbent core between the topsheet and the backsheet. Such absorbent articles are typically disposable, and are disposed of after use. The topsheet is typically a liquid-permeable sheet to allow liquid, urine etc., to enter the absorbent core; the backsheet is largely or completely liquid-impermeable to prevent leakage from the absorbent article.

In one embodiment of the present invention the backsheet may be provided with a wetness indicator. For example the wetness indicator may be a coloured strip which changes colour or shade when exposed to urine. In a most preferred embodiment the wetness indicator is a coloured strip which is printed onto the inside (i.e. body side) of the backsheet. In this embodiment the wetness indicator is in liquid communication with the absorbent core and consequently the wetness indicator is exposed to the same environment as that of the core. When an insult of urine is absorbed by the absorbent core then the wetness indicator responds. In preferred embodiments the wetness indicator responds to the presence of urine by changing colour or shade.

In a preferred embodiment the wetness indicator is a coloured strip, for example a strip of about 10 mm width, lying longitudinally along the centre line of the absorbent article. In one particularly preferred embodiment the wetness indicator comprises a pH sensitive chemical which changes colour or shade when urine (low pH) is present.

In an alternative embodiment of the present invention the wetness indicator comprises one or more sensors in liquid communication with the absorbent core which sense the presence of liquid by resistance/conduction, capacitance, impedence or by some other electrical characteristic. For example, one or more wires may be embedded in or adjacent to the absorbent core. Electrical characteristics of these wires may be detected by sensors in the wearable sensing device to indicate when liquid, for example urine, is present. Examples of sensor systems are disclosed in WO2012/166765, published on December 6th 2012.

A wearable sensing device may be reusable, and intended to be reused multiple times after multiple changes of disposale absorbent articles.

A wearable sensing device may contain one or more sensors, for example: wetness sensor(s); chemical sensor(s), including volatile organic chemical (VOC) sensor(s); accelerometer(s); temperature sensor(s); humidity sensor(s); microphone(s); gyroscope(s); magnetometer(s); global positioning sensor(s); and any of these types of sensor in combination.

In embodiments of the present invention the absorbent article comprises first and second attachment regions.

Each of the attachment regions preferably comprises mechanically interoperating elements such as outwardly facing fibres. Outwardly facing fibres in the form of "hooks" or "loops" in an attachment region interconnect with corresponding "hooks" or "loops" projecting from the cooperating attachment region. For example "hooks" or "loops" on the external housing of the wearable sensing device such as to enable the wearable sensing device to be attached to the cooperating side of the garment-facing side of the absorbent article and subsequently detached. Most preferably the garment-facing side of the absorbent article is provided with an attachment region that comprises interconnecting "hooks" or "loops".

The first attachment region is configured to secure fastening elements of the absorbent article around the waist of the wearer. The attachment between the fastening elements and the first attachment region are required to resist shear forces and providing the secure attachment of the absorbent article around the waist during normal wearing. At the same time it should be relatively easy to peel open the attachment between the fastening elements and the first attachment region in order to remove the absorbent article for disposal after use, or simply to refasten the absorbent article around the waist in case it is at first fastened incorrectly, e.g. too tightly or too loosely.

The second attachment region is configured to secure the sensing device to the second attachment region, i.e. correctly located relative to the wetness indicator, for example,or in a position where wear comfort is provided, i.e. in a position where it does not rub with legs or thighs. The attachment between the sensing device and the second attachment region are required to resist peeling forces. For example, it should be difficult, or practically impossible, for a small child to remove the sensing device from the absorbent article, whilst at the same time an adult carer should be able to apply sufficient force to remove the sensing device from the absorbent article in order to reapply to a new, clean absorbent article or simply to reposition the sensing device on the absorbent article.

The first attachment region preferably comprises a surface that is different than that of the second attachment region. The differences can be driven by their separate functions of the first and second attachment regions. And the differences can vary signficantly. For example, the difference in surface can arise from a differences in outwardly facing fibers (hooks or loops) when they are present. The size (length, diameter, etc.) and/or geometry of the hooks/loops within the first and second attachment regions can differ. The density or overall footprint of the hooks/loops within the two attachment regions can also differ. Hooks/loops in the different attachment regions can differ in flexiblity or stiffness. Hooks/loops in the different attachment regions may comprise different material make-up (for example, different polymer or polymer blends). Loops can be a knit material, brushed knit, or a non-woven. Also basis weight, fuziness, softness, dimensions, transparency differences. The opposing surface, or surface facing the absorbent article, may additionaly or alternatively differ between the first attachment region and the second attachment region. Still further, the difference between the first attachment region surface and a second attachment region surface could be in the color, tinting, or printing of the two surfaces. Yet another difference may be the one surface provides a releasble adhesive fastening while the other comprises hook and loop fastening capability. The peel force response of the hooks/loops of the first attachment region can be great than or less than the peel force response of the hooks/loops of the second attachment region. The surface area of the interface between the first surface attachment elments and its counter attachment element may be different and the surface area of the interface between the second surface attachment elments and its counter attachment element maybe different and the magnitude of these two different values will be different.

The wearable sensing device is thus attached to the outside (i.e. garment side) of the backsheet. In an embodiment comprising a wetness indicator, the wearable sensing device should be in sufficiently close proximity to the wetness indicator so that the wearable sensing device senses changes in colour of the wetness indicator. The changes of colour which may be sensed by the wearable sensing device may be imperceptible, or barely perceptible, to the human eye, or the changes of colour may also be visible to the human eye. Light should be transmitted through the backsheet and through the second attachment region sufficient to enable the wearable sensing device to sense changes, even small changes, in colour of the wetness indicator. Preferably the opacity of the combination of the backsheet and the second atachment region should be less than 80%, more preferably less than 60%.

The backsheet may be provided with backsheet graphics to indicate the optimum position for the placement of the wearable sensing device. For example an outline or profile may be indicated on the backsheet. In one embodiment the backsheet graphic is printed onto the inside (i.e. body side) of the backsheet, whilst in another embodiment the backsheet graphic may be printed onto the outside (i.e. garment side) of the the backsheet. Alternatively the second attachment region maybe provided with a graphic indication to indicate the optimum position for the placement of the wearable sensing device.

The peel strength and the shear strength of: i) the first attachment region and the fastening elements; and ii) the second attachment region and the sensing device, can be measured using test methods as set out below.

For the first attachment region and the fastening elements the shear force is preferably greater than 38N, more preferably greater than 40N. In addition the peel force may be less than 2N.

For the second attachment region and the sensing device, the peel force is preferably greater than 3N. In addition the shear force maybe less than 35N, or less than 30N.

Values for peel force and shear force are preferably determined using ASTM D5169-98 for shear strength (dynamic method) of hook and loop touch fasteners; and ASTM D5170-98 for peel strength ("T" method) of hook and loop touch fasteners. Other test methods are also described herein.

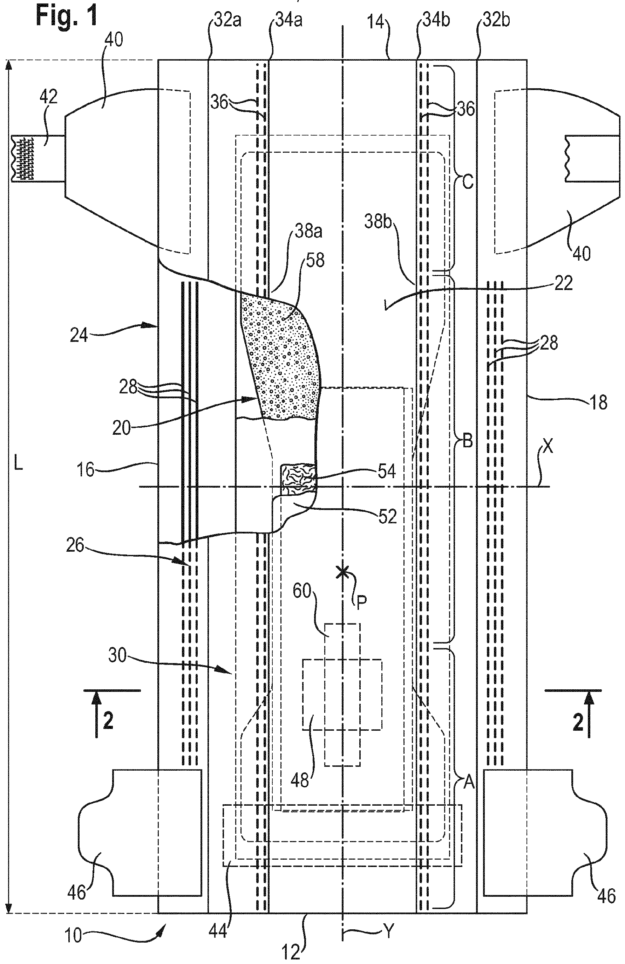

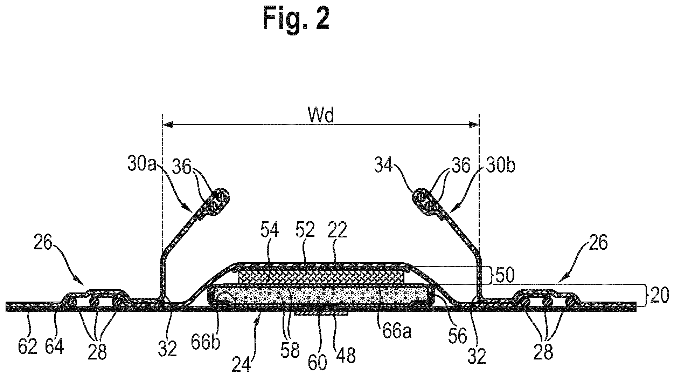

An exemplary absorbent article according to the invention in the form of a diaper 10 is represented in Figs. 1 and 2.

Fig. 1 is a plan view of the exemplary diaper 10, in a flattened state, with portions of the structure being cut-away to more clearly show the construction of the diaper 10. This diaper 10 is shown for illustration purpose only as the invention may be used for making a wide variety of diapers or other absorbent articles. The diaper extends from a front edge 12 to a longitudinally opposed rear edge 14. It comprises left side edge 16 and transversally opposed right side edge 18. The diaper 10 comprises an absorbent core 20 which is positioned between topsheet 22, which is at least partially liquid permeable and backsheet 24, which is essentially impermeable to liquid.

In Figure 1 "X" denotes a transversal access through the geometrical center of the diaper, and axis "Y" denotes the longitudinal direction. The area A denotes the front area of the diaper as seen in the longitudinal direction and C denotes the rear area of the diaper as seen in the longitudinal direction, and B denotes the central area or crotch area positioned between area A and area B, in the longitudinal direction. L denotes the length of the diaper from the front edge 12 to rear edge 14 as measured in the longitudinal direction.

The article comprises a crotch point P defined herein as the point placed on the longitudinal axis at a distance of two fifth (2/5) of L starting from the front edge 12 of the diaper 10.

The absorbent article may comprise a wetness indicator 60, which can be printed on to the backsheet. Alternatively the wetness indicator 60 may take the form of a small sheet of material or patch. As shown, a rectangular form is useful. The wetness indicator 60 can be arranged in the front area A, the central area B or the rear area C of the diaper. It is often useful to range the wetness indicator 60 in the central area B or in the front area A. As shown, it can be useful to provide the wetness indicator 60 towards the front of the crotch point P.

The diaper 10 further comprises gasketing cuffs 26 for maintaining a tight fit of the diaper 10 to the wearer, when the diaper 10 is worn. The gasketing cuffs 26 comprise elastics 28 for maintaining the tight fit, which helps to avoid leakage.

The diaper 10 further comprises barrier leg cuffs 30 on each side of the diaper. Barrier leg cuffs comprise proximal edges 32a and 32b, which are adjacent to topsheet 22. Opposed to the respective proximal edges, the barrier leg cuffs 30 comprise distal edges 34a and 34b, respectively. In the area of the distal edges 34, further elastics 36a provided, while a portion of the distal edges 34 of the barrier leg cuffs 30 can be attached to components of the diaper 10, such as the topsheet 22, it is preferred that the barrier leg cuffs 30 also comprise unattached areas of the distal edges, herein referred to as free flaps 38. The respective free flaps 38 are typically provided in the central region of the diaper 10.

The diaper 10 further comprises the fastening system, for fastening the diaper to the body of a wearer. This fastening system comprises two back ears 40, which comprise adhesive tapes 42. The adhesive tapes 42 can be attached to first attachment region 44. In the front area, the diaper comprises front ears 46. As described below, for other embodiments other fastening systems can be useful, including mechanical fasteners.

The diaper 10 further comprises second attachment region 48 for securing a wearable sensing device to the diaper.

The core can optionally comprise areas, where there is a reduced amount of absorbent material or no absorbent material. These areas are referred to as channels.

Figure 2 is transversal cross-section of the embodiment of Figure 1 and readily shows other structural elements of the diaper. As shown in this figure, the diaper comprises an acquisition-distribution system 50. This acquisition-distribution system comprises acquisition layer 52, which first receives liquid, and distribution layer 54 underneath acquisition layer 52.

The absorbent core 20 comprises a core layer 56. This core layer can comprise particular material 58, such as super absorbent particles, herein also referred to SAP. Between the core layer 56 and the backsheet 24 the wetness indicator 60 can be arranged. As shown in Fig. 2 the backsheet 24 can comprise and inner backsheet layer 62 (which is oriented towards the core 20) and an outer backsheet layer 64, which is generally oriented towards the garments of a wearer. As shown in Fig. 2, in accordance with the present invention, the wetness indicator 60 can be provided above the inner backsheet layer and below the core wrap 66, more precisely, below the portion of the core wrap 66 which is oriented towards the backsheet 24. The second attachment region 48 is provided on the outer backsheet layer 64. Preferably the second attachment region 48 is a discrete material from the backsheet 24 and the discrete second attachment region 48 is attached, for example adhesively attached, to the backsheet 24. A wearable sensing device (not shown) can be releasably affixed to the second attachment region 48 so that the wearable sensing device is correctly positioned with respect to the wetness indicator 60.

In one embodiment of the present invention the discrete second attachment region 48 is affixed to the backsheet 24 during the assembly of the absorbent article. In this embodiment the second attachment region 48 is attached in-line, i.e. by a processing step carried out as one step of the assembly of the absorbent article on a converting apparatus. For example, the second attachment region 48 may be attached by a conventional cut and slip process. In an alternative embodiment of the present invention the discrete second attachment region 48 is attached to the backsheet 24 offline, before the backsheet 24 is assembled together with the rest of the absorbent article. For example, the second attachment region 48 may be combined with inner and outer backsheet layers to form a trilaminate. The trilaminate, including the second attachment layer, may be stored, for example on a roll of material, and subsequently transported to the converting apparatus or festooned. The trilaminate is then assembled together with the rest of the absorbent article.

### Wearable Sensing Device

The wearable sensing device comprises a housing which contains various components, such as electronic components. The housing may be a rigid housing or it may be a flexible or partly flexible housing. The components contained by the housing may be selected from the list comprising: data processing unit, memory storage, power source, light emitting device, sensors, data transmission unit, antenna, output units such as indicators, display unit and some or all of the units in combination.

In one embodiment of the invention the sensor, or one of the sensors, is a color sensor which can generate an output which depends on a color observed by the color sensor. It can also be referred to as an optical sensor. A useful color sensor can comprise a photo-diode. For example color sensor TCS 34725, commercially available from AMS-TAOS USA Inc., Plano, Texas, has been found useful. The TCS 34725 device provides a digital return of red, green, blue (RGB). An IR blocking filter, integrated on-chip and localized to the color sensing photodiodes, minimizes the IR spectral component of the incoming light and allows color measurements to be made accurately.

The wearable sensing device may also comprise a light emitting device, such as an LED, for emitting light onto an area, the color of which is to be assessed by the color sensor. The color sensor is in particular optimized for assessing the color of the color-changing indicator. The color sensor might be sensitive to visible and non-visible light, namely light in the near IR range. Sensor of this type are referred to as hyperspectral sensors. As discussed above the wetness indicator can change its color, and preferably does change its color, based on the presence of bodily exudates. The color sensor, when positioned appropriately with respect to the wetness indicator, will therefore provide an output, which varies depending on the presence of bodily exudates.

The wearable sensing device may also comprise motion sensors. Suitable motion sensors may be selected from accelerometer(s), gyroscope(s), magnetometer(s), or combinations of these sensors.

The wearable sensing device may also comprise a data transmission unit which transmits data, preferably wirelessly. The skilled person is aware of useful standards for providing such data transmission, for example Bluetooth, BTLE, mesh (e.g., IEEE 802.15.4), WiFi (e.g., IEEE 802.15.11), communication incorporating all or any portion of IEEE 802 or similar communication standards, RFID, 3G, 4G, 5G communication, Backscatter communication, light communication, audio/sound communication, harvesting protocol communication (e.g., a metadata harvesting protocol). Other communications protocols or combinations of communications protocols (e.g., a Bluetooth/Mesh combined protocol) can be employed. Additionally or alternatively an acoustic or optical broadcasting is useful.

The wearable sensing device may comprise a power source which is designed to provide sufficient electrical power to operate the device for a period of time, for example up to three months, or up to six months. Alternatively, the wearable sensing device may comprise a rechargeable power source.

### Test Methods

1. Test method 1 is to determine the vertical peel force under a preset horizontal shear force. Defined pressure is applied to a hook sample and a loop sample which are brought in contact vertically. A defined horizontal shear force is applied, then the sample is removed vertically and the necessary force recorded.
2. Test method 2 is to determine the 45° peel force to detachment of hooks with a solid backing and the hook tape only. A hook sample and a loop sample with a solid backing is brought in contact vertically with a defined pressure. The support is tilted to 45 degree from the horizontal. A peel force is applied to the sample at the lower longitudinal end of the solid backing attached to the loop sample. The necessary vertical peel force required to separate the loop sample from the hook sample is recorded.

### Sample Preparation:

All testing is performed in a conditioned room maintained at about 23 °C ± 2 °C and about 50% ± 2% relative humidity. Precondition the samples at about 23 °C ± 2 °C and about 50% ± 2% relative humidity for 2 hours prior to testing.

Identify the landing zone portion for the diaper fastening tape and the landing zone for the wetness measurement device on the outer surface of the article. Remove the laminate with the wetness indicator such to leave the laminate present on the garment facing side including the landing zone intact, using cryogen freeze spray (such as CytoFreeze, Control Company, TX or equivalent). If the article does not have a distinct landing zone, cut an area situated above (garment facing) the wetness indicator from the article and remove the layer with the wetness indicator such to leave the laminate present on the garment facing side intact, using cryogen freeze spray (such as CytoFreeze, Control Company, TX or equivalent).

Prepare a tape laminate to be used to attach the aforementioned landing zone or article area to the surface 206 of the bottom fixture 200. Attach a layer of 3M 1524 Transfer Adhesive to a strip of 3M Double Coated Tape 9589. Remove the backing paper of the 3M 1524 and carefully without forming wrinkles or bubbles, attach the landing zone material, loops upwards to the 3M 1524/9589 laminate. The landing zone laminate is trimmed to approximately 51 mm corresponding to the article's lateral direction and 26 mm corresponding to the article's longitudinal direction. The landing zone sample must be dimensional equal or larger than the hooks material.

Remove the hook material from the absorbent article and the device using cryogen freeze spray. If the hook material cannot be removed without damage, cut the hook material from the article and remove as much laminate materials from the body side as possible without damaging the hook material. Prepare a tape laminate to be used to attach the hooks to the surface 102 of the upper fixture 100. Attach a layer of 3M 1524 Transfer Adhesive to a strip of 3M Double Coated Tape 9589. Remove the backing tape of the 3M 1524 and carefully without forming wrinkles or bubbles, attach the hook material, hooks upwards to the 3M 1524/9589 laminate. The hooks laminate is trimmed to 13 mm ± 0.05 mm in the direction corresponding to the article's lateral direction and 25.4 mm ± 0.05 mm in the direction corresponding to the article's longitudinal direction.

Remove the backing paper from the landing zone specimen and place it, loops side up, onto the surface 206, such that the lateral direction (with respect to the article) is oriented perpendicular to the axis of the plate 204 of the lower fixture. Place a 250 g weighted plate (surface of 50 mm x 76 mm) onto the landing zone specimen. Without applying any additional pressure slide the plate over the entire surface of landing zone specimen, to adhere it to surface 206.

Remove the backing on the hook specimen and attach the hooks to the downward facing surface 102 on the upper fixture 100. The hooks are oriented to the already mounted landing zone as they would be oriented in use on the absorbent article.

### Equipment:

A constant rate of extension tensile tester with computer interface (such as a MTS SYNERGIE 200 tensile tester, controlled with TestWorks 4 software, as available from MTS Systems Corp., Eden Prairie, Minnesota, or equivalent), fitted with an appropriate load cell is used for each of these tests. The load cell is selected to be operated within 10% and 90% of its stated maximum load.

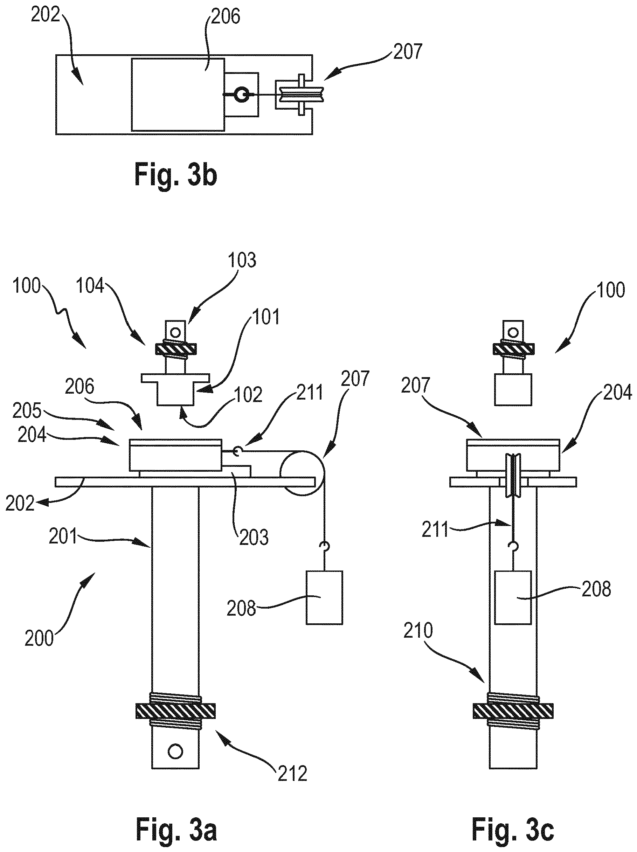

For test method 1 (vertical peel force) two custom fixtures are fabricated. Referring to Figures 3a to 3c, the first fixture 100 includes a rectangular foot 101 that attaches to the upper movable crosshead of the tensile tester and has a downward-facing planar surface 102 orthogonal to the path of travel of the cross head, onto which a hooks specimen is to be affixed. The second fixture 200 attaches to the bottom, stationary mount of the tensile tester, and consists of a support shaft 201, base 202. For test method 1 the fixture also comprises a sliding plate 204 having an upward-facing planar surface 206 orthogonal to the path of travel of the crosshead, onto which the landing zone specimen is to be affixed. Thus, when the test is performed, the loops side of the landing zone specimen is oriented facing and parallel to, the hooks side of the hooks specimen as it would be oriented on the absorbent article.

Still referring to Figures 3a and 3c, the upper fixture 100 consists of a rectangular foot 101 affixed to a suitable mounting device such as an upper mounting shaft 103 adapted to connect to the movable crosshead of the tensile tester. Upper mounting shaft 103 is threaded as shown, and has a locking collar 104. When the upper mounting shaft 103 is connected to the mount of the crosshead, the locking collar 104 is turned against the mount, to immobilize the fixture 100 relative the crosshead, such that it will move integrally along with the crosshead during testing, without any interplay therebetween. The foot 101 is formed of aluminum with a downward-facing, planar, surface 102 orthogonal to the path of travel of the crosshead. The downward-facing surface 102 must be of sufficient length and width to accept the entirety of a hooks specimen. The shorter side is perpendicular to the axis of the plate 204 of the lower fixture, and must be substantially centered about the axis of upper mounting shaft 103. The top edge of the foot 101, forms a "T" perpendicular to the axis of the plate 204 of the lower fixture , to aid the analyst while mounting the hooks specimen.

Referring to Figures 3a to 3c, the lower fixture 200 consists of a support shaft 201 with two horizontal support plates 202 and 203. The lower end of the support shaft 201 is designed to fit within the bottom mount of the tensile tester and is threaded as shown. When the support shaft 201 is connected to the base of the tensile tester, a locking collar 212 is turned against the mount, to immobilize fixture 200 and align it vertically with the top fixture 100. A rectilinear plate 205 is attached to the slide 204, with an upward-facing, planar, surface 206, orthogonal to the path of travel of the crosshead. The upward-facing surface 206 must be of sufficient length and width to accept the entirety of a landing zone specimen.

In addition, for Test Method 1, a ball bearing slide 204 (40 x 90 x 6 mm aluminum plate, 30mm travel) (McMaster Carr, Robbinsville, NJ, Part No. 6257K28 or equivalent) is mounted on the upper support plate 202 such that when the slide 204 is at its starting position, it is centered under the upper fixture 100. The slide 204 allows horizontal, free movement along its longitudinal axis. The upper surface 102 and the lower surface 206 of the two fixtures are parallel with a parallelism tolerance of no more than 0.01 mm. The right end of the upper support plate 202 is forked to mount a low friction wheel 207 (see Fig. 3b). The wheel 207 translates a vertical force from a suspended weight 208 to a horizontal force applied to the slide 204 via a flexible 1mm diameter braided steel wire 211 that is connected to the slide, threaded overtop the wheel and attached to the weight. The weight 208 is removable so that different weights can be applied depending on the desired force.

### Test Method 1:

The gauge between the downward-facing surface 102 and the upward-facing surface 206 is set to 50 mm. Program the tensile tester to move the upper fixture 100 down at 5 mm/s for 49 mm, and then continue downward at 0.5 mm/s until the target application force is detected at the load cell. Within 5 sec. of achieving the target application force, attach the weight 208 to the wire. Within 5 sec. after the weight 208 is suspended, start data collection as the upper fixture is raised at 5.0 mm/sec. until the hooks and loops have become disengaged.

Position the slide 204 such that the landing zone specimen is centered underneath the hook specimen. Zero the load cell and crosshead position, and start the test. The energy to detachment (mJ) (area under the curve from zero force to detachment) is collected and recorded to the nearest 0.01 mJ.

A total of six combinations in 5 replicates each, absorbent product hooks on device landing zone, diaper landing zone and non-woven backsheet as well as the device hooks on device landing zone, diaper landing zone and non-woven backsheet, are tested with 1.00 N application force and 700g shear weight. If the specimen lacks some of the aforementioned areas the number of test combinations is reduced accordingly.

For each of six abovementioned combinations, the arithmetic mean of the energy of detachment (area under the curve from zero force to detachment of five replicates in mJ is calculated and reported to the nearest 0.01 m

### Test Method 2:

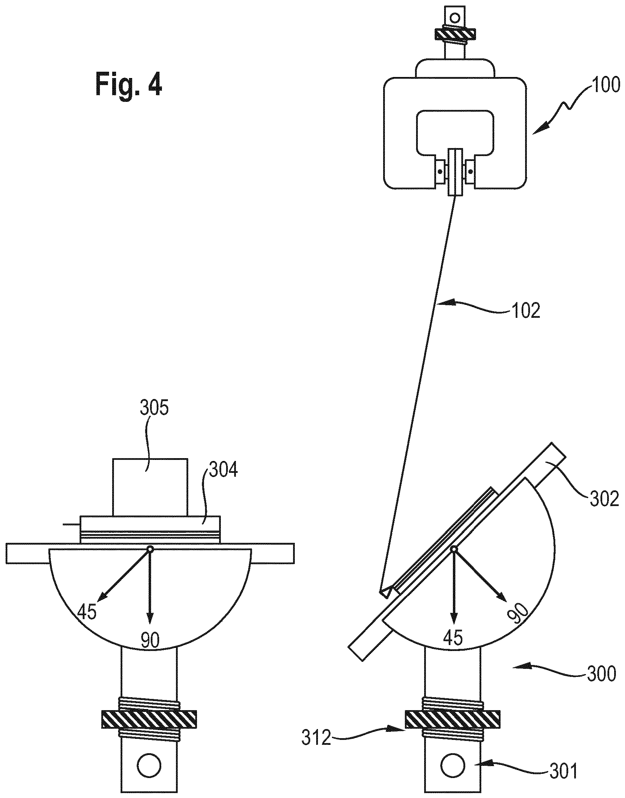

For this test, two custom fixtures are fabricated. Referring to Figure 4 the first fixture 100 includes steel wire 102, that attaches to the upper movable crosshead of the tensile tester with a hook or clip at the lower end, to which the specimen is to be affixed. the lower fixture 300 consists of a support shaft 301 with a horizontal support plates 302. The lower end of the support shaft 301 is designed to fit within the bottom mount of the tensile tester and is threaded as shown. When the support shaft 301 is connected to the base of the tensile tester, a locking collar 312 is turned against the mount, to immobilize fixture 300 and align it vertically with the top fixture 100. The rectilinear plate 302 with an upward-facing, planar, surface is movable from the horizontal to 45 degrees, the measurement is done at 45 degrees, whereas the sample is attached to the landing zone in the horizontal position, to allow a uniform attachment force via a 300g weight 305 being placed on the top of the hook tape or the polymer support 304.

### Sample Preparation:

Identify the landing zone portion for the diaper fastening tape and the landing zone for the wetness measurement device on the outer surface of the article. Remove the laminate with the wetness indicator such to leave the laminate present on the garment facing side including the landing zone intact, using cryogen freeze spray (such as CytoFreeze, Control Company, TX or equivalent). If the article does not have a distinct landing zone, cut an area situated above (garment facing) the wetness indicator from the article and remove the layer with the wetness indicator such to leave the laminate present on the garment facing side intact, using cryogen freeze spray (such as CytoFreeze, Control Company, TX or equivalent).

Prepare a tape laminate to be used to attach the aforementioned landing zone or article area to the surface 206 of the bottom fixture 200. Attach a layer of 3M 1524 Transfer Adhesive to a strip of 3M Double Coated Tape 9589. Remove the backing paper of the 3M 1524 and carefully without forming wrinkles or bubbles, attach the landing zone material, loops upwards to the 3M 1524/9589 laminate. The landing zone laminate is trimmed to approximately 51 mm corresponding to the article's lateral direction and 26 mm corresponding to the article's longitudinal direction. The landing zone sample must be dimensional equal or larger than the hooks material.

Remove the hook material from the absorbent article and the device using cryogen freeze spray. If the hook material cannot be removed without damage, cut the hook material from the article and remove as much layer materials from the body side as possible without damaging the hook material. Attach a layer of 3M 1524 Transfer Adhesive to a strip of 3M Double Coated Tape 9589. Remove the backing tape of the 3M 1524 and carefully without forming wrinkles or bubbles, attach the hook material, hooks upwards to the 3M 1524/9589 laminate. The hooks laminate is trimmed to 13 mm ± 0.05 mm in the direction corresponding to the article's lateral direction and 25.4 mm ± 0.05 mm in the direction corresponding to the article's longitudinal direction.

For sample placement position the Plate 302 in a 90 angle to the shaft 301. Remove the backing paper from the landing zone specimen and place it, loops side up, onto the surface 302, such that the lateral direction (with respect to the article) is oriented perpendicular to the short axis of plate 302.

Remove the backing on the hook specimen and attach the hooks to a polymer plate 304 (50 mm x 76 mm x 10 mm) with a eyelet at a shorter end. For a second test use the loop material without an adhesive backing. Attach the hook material to the loops. The hooks should be oriented to the already mounted landing zone as they were oriented on the absorbent article in use. Gentle place a 300 g weighted plate 305 onto the hook loop laminate to assure a consistent hook loop engagement.

For testing position the plate 302 at a 45 degree angle to shaft 301. Attach wire 102 to the eyelet on the polymer plate at the lower end of the plate. For the second test use a paperclip or equivalent to attach wire 102 to the lower end of the hook tape sample.

Program the tensile tester to move the upper fixture 100 up at 305 mm/s for 120mm, and start data collection until the hooks and loops have become disengaged. The gage between the upper fixture 101 and the upward-facing surface 202 is set to 130 mm. Record and report the Peak load (N) to the nearest 0.01 N.

Program the tensile tester to move the upper fixture 100 up at 305 mm/s for 120mm, and start data collection until the hooks and loops have become disengaged. The gage between the upper fixture 101 and the upward-facing surface 302 is set to 130 mm. Record and report the Peak load (N) to the nearest 0.01 N.

### Test Results:

### Test Method 1

1 N engagement force, vertical pull with horizontal shear 7N, energy to detachment mJ

| | First Attachment Region | Second Attachment Region |
|---|---|---|
| Fastening elements | 40.2 | |
| Sensor Device | | 35.8 |

### Test Method 2

1 N engagement force, vertical pull, N

| | First Attachment Region | Second Attachment Region |
|---|---|---|
| Fastening Elements | 2.3 | |
| Sensor Device | | 5.5 |

Other test methods which may be suitable for assessing hook and loop materials include:
ASTM D5169-98, a standard test method for shear strength (dynamic method) of hook and loop touch fasteners;
ASTM D5170-98, a standard test method for peel strength ("T" method) of hook and loop touch fasteners.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An absorbent article comprising topsheet (22), backsheet (24) and an absorbent core (20) between the topsheet (22) and the backsheet (24), the article comprising a front waist region, a back waist region, and a central region between the front and back waist regions, and further comprising fastening elements for securing the article around the waist of a wearer, wherein the article comprises at least two attachment regions (44, 48):
a first attachment region (44) for securing the fastening elements in the waist region, wherein the first attachment region (44) lies in at least a part of the front waist region, the first attachment region (44) comprises first surface comprising outwardly facing fibres, wherein the fastening elements comprise a fastening surface comprising outwardly facing fibres, and wherein the outwardly facing fibres interact to form a releaseable attachment between the fastenening elements and the first attachment region, wherein the interacting outwardly facing fibres comprise hooks and loops; and
a second attachment region (48) for securing a wearable sensing device, the second attachment region (48) being located on the side of the backsheet (24) opposite to the absorbent core (20), wherein the second attachment region (48) lies in at least a part of the central region between the front and back waist regions and wherein the second attachment region (48) comprises hooks and/or loops;
the first attachment region (44) has a first surface and the second attachment region (48) has a second surface and wherein the first surface is different from the second surface, and **characterised in that** the peel force response of the hooks/loops of the first attachment region is greater than or less than the peel force response of the hooks/loops of the second attachment region.

2. The absorbent article according to Claim 1, wherein the absorbent article further comprises a wetness indicator (60), the wetness indicator (60) being in liquid communication with the absorbent core (20) on one side of the backsheet (24), and wherein the second attachment region (48) overlies at least part of the wetness indicator (60) on the opposing side of the backsheet (24).

3. The absorbent article according to Claim 2, wherein the article comprises a graphic indication for the placement of a wearable sensing device at least in part overlying the wetness indicator (60).

4. The absorbent article according to Claim 3 wherein the graphic indication is printed on to the backsheet (22).

5. The absorbent article according to Claim 3 wherein the graphic indication is printed on to the second attachment region (48).

6. A system comprising an absorbent article according to any of the previous Claims, and a wearable sensing device, wherein the wearable sensing device comprises a housing, and wherein a part of the housing cooperates with the second attachment region (48) to releasably secure the wearable sensing device to the absorbent article.

7. The system according to Claim 6 wherein the housing is provided with outwardly facing fibres which interact to form a releaseable attachment between the housing of the wearable sensing device and the second attachment region (48).

## Patentansprüche

1. Absorptionsartikel, umfassend eine Deckschicht (22), Unterschicht (24) und einen Absorptionskern (20) zwischen der Deckschicht (22) und der Unterschicht (24), wobei der Artikel einen vorderseitigen Taillenbereich, einen rückseitigen Taillenbereich und einen zentralen Bereich zwischen dem vorderseitigen und dem rückseitigen Taillenbereich umfasst und ferner Befestigungselemente zum Sichern des Artikels um die Taille eines Trägers umfasst, wobei der Artikel wenigstens zwei Befestigungsbereiche (44, 48) umfasst:
einen ersten Befestigungsbereich (44) zum Sichern der Befestigungselemente im Taillenbereich, wobei der erste Befestigungsbereich (44) in wenigstens einem Teil des vorderseitigen Taillenbereichs liegt, der erste Befestigungsbereich (44) eine erste Oberfläche umfasst, die nach außen weisende Fasern umfasst, wobei die Befestigungselemente eine Befestigungsoberfläche umfassen, die nach außen weisende Fasern umfasst, und wobei die nach außen weisenden Fasern zusammenwirken, um eine lösbare Befestigung zwischen den Befestigungselementen und dem ersten Befestigungsbereich zu bilden, wobei die zusammenwirkenden, nach außen gerichteten Fasern Haken und Schlaufen umfassen; und
einen zweiten Befestigungsbereich (48) zum Befestigen eines tragbaren Detektorelements, wobei der zweite Befestigungsbereich (48) auf der Seite der Unterschicht (24) gegenüber dem Absorptionskern (20) angeordnet ist, wobei der zweite Befestigungsbereich (48) in wenigstens einem Teil des zentralen Bereichs zwischen dem vorderseitigen und dem rückseitigen Taillenbereich liegt und wobei der zweite Befestigungsbereich (48) Haken und/oder Schlaufen umfasst;
wobei der erste Befestigungsbereich (44) eine erste Oberfläche und der zweite Befestigungsbereich (48) eine zweite Oberfläche aufweist und wobei sich die erste Oberfläche von der zweiten Oberfläche unterscheidet, und
**dadurch gekennzeichnet, dass** die Schälkraftreaktion der Haken/Schlaufen des ersten Befestigungsbereichs größer oder kleiner als die Schälkraftreaktion der Haken/Schlaufen des zweiten Befestigungsbereichs ist.

2. Absorptionsartikel nach Anspruch 1, wobei der Absorptionsartikel ferner einen Feuchtigkeitsindikator (60) umfasst, wobei der Feuchtigkeitsindikator (60) in flüssiger Verbindung mit dem Absorptionskern (20) auf einer Seite der Unterschicht (24) ist und wobei der zweite Befestigungsbereich (48) über wenigstens einem Teil des Feuchtigkeitsindikators (60) auf der gegenüberliegenden Seite der Unterschicht (24) liegt.

3. Absorptionsartikel nach Anspruch 2, wobei der Artikel eine grafische Anzeige für die Platzierung eines tragbaren Detektorelements umfasst, die wenigstens teilweise über dem Feuchtigkeitsindikator (60) liegt.

4. Absorptionsartikel nach Anspruch 3, wobei die grafische Anzeige auf die Unterschicht (22) gedruckt ist.

5. Absorptionsartikel nach Anspruch 3, wobei die grafische Anzeige auf den zweiten Befestigungsbereich (48) gedruckt ist.

6. System, umfassend einen Absorptionsartikel nach einem der vorstehenden Ansprüche und ein tragbares Detektorelement, wobei das tragbare Detektorelement ein Gehäuse umfasst und wobei ein Teil des Gehäuses mit dem zweiten Befestigungsbereich (48) zusammenwirkt, um das tragbare Detektorelement an dem Absorptionsartikel zu sichern.

7. System nach Anspruch 6, wobei das Gehäuse mit nach außen gerichteten Fasern versehen ist, die zusammenwirken, um eine lösbare Befestigung zwischen dem Gehäuse der tragbaren Erfassungsvorrichtung und dem zweiten Befestigungsbereich (48) zu bilden.

## Revendications

1. Article absorbant comprenant une feuille de dessus (22), une feuille de fond (24) et une âme absorbante (20) entre la feuille de dessus (22) et la feuille de fond (24), l'article comprenant une région de taille avant, une région de taille arrière, et une région centrale entre les régions de taille avant et arrière, et comprenant en outre des éléments d'attache pour fixer l'article autour de la taille d'un porteur, dans lequel l'article comprend au moins deux régions d'attachement (44, 48) :
une première région d'attachement (44) pour fixer les éléments d'attache dans la région de ceinture, dans lequel la première région d'attachement (44) se trouve dans au moins une partie de la région de taille avant, la première région d'attachement (44) comprend une première surface comprenant des fibres faisant face vers l'extérieur, dans lequel les éléments d'attache comprennent une surface d'attache comprenant des fibres faisant face vers l'extérieur, et dans lequel les fibres faisant face vers l'extérieur interagissent pour former un attachement libérable entre les éléments d'attache et la première région d'attachement, dans lequel les fibres faisant face vers l'extérieur interagissant comprennent des crochets et boucles ; et
une deuxième région d'attachement (48) pour fixer un dispositif de détection portable, la deuxième région d'attachement (48) se situant sur le côté de la feuille de fond (24) opposé à l'âme absorbante (20), dans lequel la deuxième région d'attachement (48) se trouve dans au moins une partie de la région centrale entre les régions de taille avant et arrière et dans lequel la deuxième région d'attachement (48) comprend des crochets et/ou boucles ;
la première région d'attachement (44) a une première surface et la deuxième région d'attachement (48) a une deuxième surface et dans lequel la première surface est différente de la deuxième surface, et **caractérisé en ce que** la réponse à une force de pelage des crochets/boucles de la première région d'attachement est supérieure ou inférieure à la réponse à une force de pelage des crochets/boucles de la deuxième région d'attachement.

2. Article absorbant selon la revendication 1, dans lequel l'article absorbant comprend en outre un indicateur d'humidité (60), l'indicateur d'humidité (60) étant en communication liquide avec l'âme absorbante (20) sur un côté de la feuille de fond (24), et dans lequel la deuxième région d'attachement (48) recouvre au moins une partie de l'indicateur d'humidité (60) sur le côté opposé de la feuille de fond (24).

3. Article absorbant selon la revendication 2, dans lequel l'article comprend une indication graphique pour la mise en place d'un dispositif de détection portable recouvrant au moins en partie l'indicateur d'humidité (60).

4. Article absorbant selon la revendication 3 dans lequel l'indication graphique est imprimée sur la feuille de fond (22).

5. Article absorbant selon la revendication 3 dans lequel l'indication graphique est imprimée sur la deuxième région d'attachement (48).

6. Système comprenant un article absorbant selon l'une quelconque des revendications précédentes, et un dispositif de détection portable, dans lequel le dispositif de détection portable comprend un logement, et dans lequel une partie du logement coopère avec la deuxième région d'attachement (48) pour fixer de manière libérable le dispositif de détection portable à l'article absorbant.

7. Système selon la revendication 6 dans lequel le logement est pourvu de fibres faisant face vers l'extérieur qui interagissent pour former un attachement libérable entre le logement du dispositif de détection portable et la deuxième région d'attachement (48).
